# EUROPÄISCHE PATENTANMELDUNG

(11) **EP 2 514 318 A1**
(43) Veröffentlichungstag der Anmeldung: **24.10.2012**
(21) Anmeldenummer: 12164984.2
(22) Anmeldetag: 20.04.2012
(51) Int. Cl.: A23K 1/00, A23K 1/14, C12M 1/113

(54) **Verwendung eines proteinreichen fettarmen pflanzlichen Produktes und Anlage und Verfahren zur Herstellung eines proteinreichen fettarmen pflanzlichen Produktes aus einem Presskuchen**

(30) Priorität: 21.04.2011 DE 102011002243; 20.01.2012 DE 102012100466
(71) Anmelder: Hochschule Fulda, 36039 Fulda (DE)
(72) Erfinder: Ahlert, Burkhard, 30974 Wennigsen (DE); Lücke, Friedrich-Karl, 37124 Rosdorf (DE)
(74) Vertreter: Kleine, Hubertus

(57) **Zusammenfassung**

Die Erfindung betrifft eine Verwendung eines proteinreichen, pflanzlichen fettarmen Produktes, welches durch Fermentation aus von einer Rapsölgewinnung erhaltenen Rückständen gewonnen wird, wobei die Rückstände aus der Rapsölgewinnung vorzugsweise aus geschältem Raps stammen, als Tierfuttermittel oder Tierfuttermittelkomponente. Weiter betrifft die Erfindung eine Anlage zur kontinuierlichen Herstellung eines proteinreichen fettarmen pflanzlichen Produktes aus einem Presskuchen, welche eine Fermenteranlage aufweist, wobei die Fermenteranlage eine Transporteinrichtung (4) für einen Transport eines zu fermentierenden Zwischenproduktes aufweist und eine Klimakammer (5) zur Fermentation, sowie ein Verfahren zur Herstellung eines proteinreichen fettarmen pflanzlichen Produktes.

## Beschreibung

Die Erfindung betrifft eine Verwendung eines proteinreichen, fettarmen pflanzlichen Produktes, eine Anlage zur kontinuierlichen Herstellung eines proteinreichen fettarmen pflanzlichen Produktes nach dem Oberbegriff des Anspruchs 3 und ein Verfahren nach dem Oberbegriff des Anspruches 8.

Als proteinreiches Lebensmittel sind z. B. Sojabohnen bekannt, die vor allem in vielen asiatischen Ländern sowie in Nord- und Südamerika angebaut werden. Zur Verbesserung des Geschmacks werden Sojabohnen zu zahlreichen Produkten wie z. B. Sojamilch, Tofu, Miso oder Tempeh verarbeitet. Die Herstellung von Tempeh erfolgt z. B. durch Fermentation unter Anwendung von Schimmelpilz-Kulturen (z. B. US Patentschriften 3,228,773 und 3,243,301). Außerdem ist es bekannt, mit Hilfe verschiedener Rezepturen zahlreiche Speisen aus diesen Produkten zuzubereiten (z. B. US-Patentschrift 4,151,307). Schließlich wird aus zerschroteten Sojabohnen durch Pressen und/oder Extraktion Sojaöl gewonnen. Die dabei erhaltenen Rückstände, meistens als Presskuchen bezeichnet, werden als Viehfutter verwendet.

Es ist außerdem bereits bekannt, statt Sojabohnen die bei der Gewinnung von Rapsöl erhaltenen Rückstände zur Herstellung von proteinreichen, pflanzlichen Lebensmitteln zu verwenden und unerwünschte Substanzen durch Fermentierung der Rückstände mit Schimmelpilzen abzubauen. (ROZAN et al in "Detoxication of rapeseed meal by Rhizopus oligosporus Sp-T3; A first step towards rapeseed protein concentrate", INTERNATIONAL JOURNAL OF FOOD SCIENCE AND TECHNOLOGY, Bd. 31; Nr. 1, 1996, Seiten 85-90; PAL VIG und WALIA in "Beneficial effects of Rhizopus oligosporus fermentation on reduction of glucosinolates, fibre and phytic acid in rapeseed (Brassica napus) meal", BIORESOURCE TECHNOLOGY , Bd. 78, Nr. 3 Juli 2001, Seiten 309-312). Auch die Herstellung von Proteinisolat durch übliche Extraktionsverfahren ist bekannt (Murray et al in "Rapeseed a potential global source of high quality plant protein", ASIAPACIFIC FOOD INDUSTRY, AP TRADE PUBLICATIONS, SINGAPORE; SG, April 2001 (2001-04), Seiten 30-34).

Die EP 1 611 800 B1 beschreibt ein proteinreiches, pflanzliches Lebensmittel, welches ein fettarmes Fermentationsprodukt aus durch die Gewinnung von Rapsöl erhaltenen Rückständen enthält oder aus diesem besteht. Dieses stammt aus Rückständen aus der Rapsölgewinnung mit geschältem Raps.

Die vorliegende Erfindung hat sich die Aufgabe gestellt, ein weiter optimiertes Verfahren, sowie eine Anlage zur Herstellung eines solchen Produktes bereitzustellen und das Anwendungsspektrum dieses Produktes auf eine neue, bislang unbekannte Verwendung zu erweitern.

Die Erfindung löst diese Aufgabe durch eine Verwendung gemäß Anspruch 1, eine Anlage mit den Merkmalen von Anspruch 3 und ein Verfahren mit den Merkmalen von Anspruch 8.

Erfindungsgemäß wird ein proteinreiches, pflanzliches fettarmes Produkt, welches durch Fermentation aus von einer Rapsölgewinnung erhaltenen Rückständen gewonnen wird, wobei die Rückstände aus der Rapsölgewinnung mit geschältem Raps stammen, als Tierfuttermittel verwendet.

Die vorliegende Erfindung beschreibt ein mit Sojaprodukten vergleichbares proteinhaltiges Tierfuttermittel aus Raps, der beispielsweise im europäischen Bereich und in Deutschland angebaut wird. Dabei wird vor allem ein Tierfuttermittel geschaffen, das einen hohen Proteingehalt hat und eine hohe biologische Wertigkeit aufweist. Im Übrigen ist das Produkt möglichst fettarm und weist einen geringen Gehalt an unerwünschten Bestandteilen auf.

Das proteinreiche Futtermittel besteht dabei ganz oder teilweise aus einem fettarmen Fermentationsprodukt, das aus den bei der Gewinnung mit Rapsöl aus geschältem oder ungeschältem Raps anfallenden Rückständen hergestellt ist. Bevorzugt werden solche Rückstände eingesetzt, deren Ölgehalt durch einen Extraktionsvorgang mit einem organischen Lösungsmittel verringert ist. Als Lösungsmittel kann insbesondere Hexan Verwendung finden.

Die Erfindung macht sich einerseits die Tatsache zu Nutze, dass Raps auch in heimischen Gegenden immer häufiger insbesondere zu dem Zweck angebaut wird, der Ernährung dienendes Rapsöl zu erzeugen oder das erhaltene Rapsöl zu technischem Öl, insbesondere Biodiesel, weiterzuverarbeiten. Andererseits schlägt die Erfindung vor, zur Herstellung des Futtermittels nicht die ganze Frucht, sondern lediglich die bei der Ölherstellung erhaltenen Rückstände bzw. den Presskuchen zu verwenden. Dadurch wird anders als bei der Verarbeitung von Sojabohnen ein weitgehend von seinen Ölbestandteilen befreites und damit fettarmes Produkt erhalten, das zwar gegenüber dem Ausgangsprodukt Raps in hohem Masse energiereduziert ist, sich im übrigen aber wie die Frucht selbst durch einen hohen Gehalt an Proteinen und anderen vorteilhaften Inhaltsstoffen auszeichnet. Das erfindungsgemäße Futtermittel ist daher für solche Tiere besonders gut geeignet, die eine fettarme, pflanzliche Ernährung benötigen. Außerdem ergibt sich der Vorteil, dass bei der Ölherstellung keine Überproduktion an Rückständen zu befürchten ist, die Rückstände vielmehr einer sinnvollen Anwendung zugeführt werden.

Üblicherweise weist ein Presskuchen aus Raps einen Glucosinolatgehalt von 15 mmol/kg Trockenmasse auf. Der Rohfasergehalt des Rapspresskuchens liegt bei 13 Gew.%. Der Anteil an ethanollöslichen Kohlenhydraten in Rapspresskuchen beträgt üblicherweise 90 g/kg.

Durch Fermentation konnte der Glucosinolatgehalt bislang um bis zu 43 % und der Rohfasergehalt um bis zu 26 % vermindert werden (PAL VIG et al. 2001). Zudem konnten die ethanollöslichen Kohlenhydrate um mehr als 80 % (ROZAN et al. 1996) vermindert werden.

Durch das erfindungsgemäße Verfahren kann der Glucosinolatanteil erstmals auf 2 mmol/kg oder weniger verringert werden, was einer Verminderung des Glucosinolatanteils gegenüber dem des Rapspresskuchens von mindestens 87% entspricht.

Zugleich konnte der Rohfasergehalt auf unter 8 Gew.% bezogen auf Trockenmasse verringert werden, was einer Verminderung gegenüber dem Rohfasergehalt des Rapspresskuchens von mindestens 32 % entspricht.

Der Gehalt an ethanollöslichen Kohlenhydraten konnte auf unter 10 g/kg Trockenmasse gesenkt werden, was einer Verminderung gegenüber dem Gehalt an ethanollöslichen Kohlenhydraten des Rapspresskuchens von mindestens 90 % entspricht

Diese Verringerung der Anteile an Glucosinolat, Rohfasern und ethanollöslichen Kohlenhydraten ermöglicht es, den Rapsanteil im Futter zu erhöhen.

Ein besonderer Vorteil des Fermentationsproduktes liegt in seinem veränderten Fett- und Eiweißgehalt gegenüber der Ausgangssaat. Während ungeschälte Rapssaat normalerweise einen Fettgehalt von etwa Gew. 40 % bis Gew. 45 % und einen Proteingehalt von etwa 20 Gew. % hat, sinkt der Ölgehalt nach einer ersten Pressung während der Ölgewinnung auf ca. 15 Gew. % ab, während der Proteingehalt im Wesentlichen unverändert bleibt oder sogar leicht ansteigt. Durch den zuvor genannten Extraktionsvorgang mit einem organischen Lösungsmittel, der ggf. in Verbindung mit einer vorausgehenden zweiten Pressung durchgeführt wird, kann der Ölgehalt bis unter 3% verringert werden. Nicht erwünschte, die Qualität des Fermentationsprodukts mindernde Bestandteile können zumindest teilweise durch vorheriges Schälen der Rapssaat entfernt werden.

Erfindungsgemäß weist eine Anlage zur kontinuierlichen Herstellung eines proteinreichen fettarmen pflanzlichen Produktes aus einem Presskuchen, eine Fermenteranlage auf, wobei die Fermenteranlage eine Transporteinrichtung für einen Transport eines zu fermentierenden Zwischenproduktes während der Fermentation aufweist und eine Klimakammer zur Fermentation.

Ein Zwischenprodukt ist in diesem Zusammenhang ein Presskuchen, welcher in Vorbereitung auf eine Fermentation mit Schimmelpilzkulturen angeimpft ist.

Durch die Transporteinrichtung wird ein kontinuierlicher Transport des Zwischenproduktes während der Fermentation durch die Klimakammer ermöglicht. Dadurch wird ein kontinuierlicher Betrieb der Anlage insgesamt ermöglicht und somit eine Erhöhung der Produktionsleistung der Anlage. So kann beispielsweise eine kontinuierliche Abfüllung des Produktes erfolgen. Die kontinuierliche Betriebsweise ermöglicht eine optimale Auslastung der Anlage durch Dauerbetrieb. Damit kann das anmeldungsgemäße Verfahren auch für eine Verwendung des fettarmen Produkts als Tierfuttermittel wirtschaftlich eingesetzt werden.

Weitere vorteilhafte Ausgestaltungen der Anlage sind Gegenstand der Unteransprüche.

Um eine besseren Durchlüftung des Zwischenproduktes während der Fermentation zu erreichen, weist die Anlage eine Vorrichtung mit Mitteln zur Komprimierung und/oder Formgebung eines zu fermentierenden Zwischenproduktes auf.

Für einen sparsamen Umgang mit Schimmelpilz-Starterkulturen zu ermöglichen, weist die Anlage eine Rückführung auf, zum Rückführen einer Teilmenge des Produktes zu einer Mischvorrichtung, in welcher die Schimmelpilzkulturen zu dem Presskuchen zugegeben werden, wodurch die Bildung des Zwischenproduktes erfolgt.

Es ist von Vorteil, wenn die Transporteinrichtung eine kontinuierliche Vorschubgeschwindigkeit aufweist. Dabei sollte die Vorschub- bzw. Transportgeschwindigkeit allerdings nur so hoch gewählt werden, dass eine möglichst vollständige Fermentation erfolgt. Dabei haben sich pro Meter Förderbandlänge Vorschubgeschwindigkeiten der Transportvorrichtung von 0,1-1 m pro Tag als besonders günstig erwiesen.

Erfindungsgemäß umfasst ein Verfahren zur Herstellung eines proteinreichen fettarmen pflanzlichen Produktes aus einem Presskuchen, die Schritte des Zerkleinerns, Anfeuchtens, Einstellens des pH-Wertes, des Animpfens mit einer Schimmelpilzkultur und die anschließende Fermentation, wobei die Fermentationszeit dadurch verringert oder die Qualität des fermentierten Produktes dadurch verbessert werden kann, indem nach der Bildung des Zwischenproduktes und vor dem Fermentieren eine Formgebung des Zwischenproduktes erfolgt. Dadurch liegt das Zwischenprodukt nun während des Fermentierens nicht mehr als Schicht vor, sondern bietet mehr Oberfläche für Sauerstoff im Rahmen der aeroben Fermentation an.

Es ist von Vorteil, wenn vor dem Animpfen des Presskuchens eine Pasteurisierung des Presskuchens erfolgt, so dass Fremdkulturen nicht die anschließende Fermentation beeinflussen und die Qualität des Produktes mindern.

Methionin ist für den Mensch und viele Nutztiere eine essentielle Aminosäure und wird vom Körper auch zur Bildung der Aminosäure Cystein benötigt. Methionin muss daher mit der Nahrung bzw. mit dem Futter aufgenommen werden. Für einen erhöhten Nährwert und die Verwendung als Futtermittel ist es daher von Vorteil, wenn das Produkt neben Proteinen auch mit Vitaminen der Gruppe B und/oder Methionin angereichert ist. Dies kann erreicht werden, indem vor der Fermentation ein Zusatz einer weiteren Starterkultur zur Anreicherung des Produktes mit Vitaminen der Gruppe B und/oder Methionin erfolgt.

In einer bevorzugten Weiterbildung des Verfahrens wird der Ölgehalt des Presskuchens durch einen Extraktionsvorgang mit einem organischen Lösungsmittel, insbesondere Hexan, verringert. Dieses kann vor oder nach dem Schritt des Zerkleinerns erfolgen. Eine derartige Reduzierung des Ölgehalts führt zu einer weiteren Verringerung des analytischen Fettgehalts des gewonnen Produkts. Zudem stammt der als Ausgangsstoff für das erfindungsgemäße Verfahren eingesetzte Presskuchen üblicherweise aus einem vorgelagerten Verfahren zur Ölgewinnung. Durch das bei der Extraktion anfallende Öl steigt die Ausbeute eines solchen Ölgewinnungsprozesses.

Das Verfahren kann sowohl im Zusammenhang mit der vorbeschrieben Anlage zur kontinuierlichen Fermentation verwendet werden, als auch bei einer chargenweisen Fermentation. Dieses gilt ebenso für die Ausgestaltung des Verfahrens, bei der der Ölgehalt des Presskuchens durch den Extraktionsprozess verringert ist.

Die vorliegende Erfindung wird anhand eines Ausführungsbeispiels und mittels der beigefügten Figur näher erläutert.

Es zeigt:

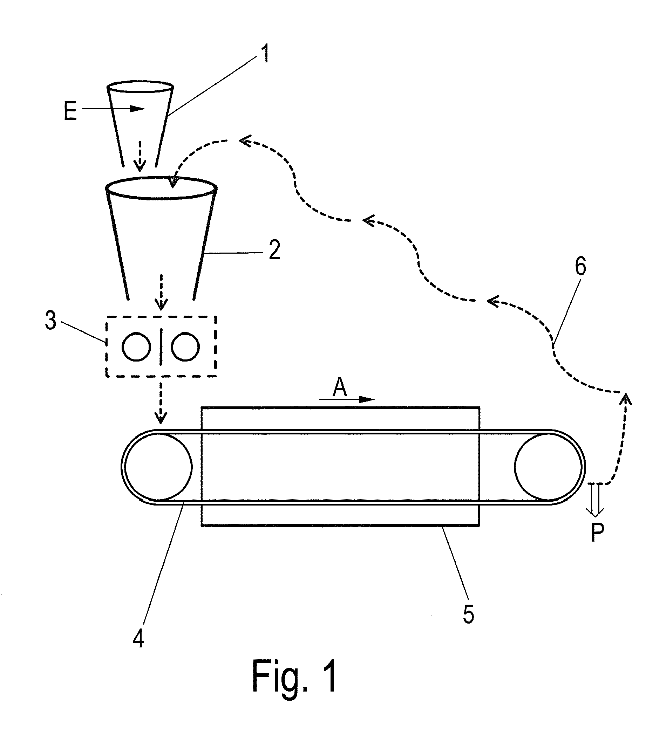

- Fig.1: den schematischen Aufbau einer erfindungsgemäßen Anlage.

Fig.1 zeigt den schematischen Aufbau einer erfindungsgemäßen Anlage zur kontinuierlichen Herstellung eines proteinreichen pflanzlichen Produktes

Nach dem Abpressen von Rapsöl in einer hier nicht dargestellten Ölpresse bleibt ein Presskuchen zurück, welcher in der oben genannten Anlage weiterverarbeitet wird.

Von der Ölpresse wird der Presskuchen über eine Zerkleinerungsvorrichtung 1 in eine Mischvorrichtung 2 überführt. In dieser Mischvorrichtung wird dem Presskuchen eine oder mehrere Schimmelpilz-Starterkulturen, die Schimmelpilz aufweisen, zugesetzt. Alternativ oder zusätzlich werden dem Presskuchen weitere Mikroorganismen, vorzugsweise Hefen, insbesondere Saccharomyces cerevisiae, die während der Fermentation Vitamine und/oder Methionin bilden, zugesetzt. Das auf diese Weise mit Kulturen angereicherte Gemisch wird im Rahmen der Anmeldung als Zwischenprodukt bezeichnet.

Von der Mischvorrichtung wird das Zwischenprodukt zu einer Vorrichtung 3 mit Mitteln zur Komprimierung und/oder Formgebung des Zwischenproduktes überführt. Eine derartige Anlage kann beispielweise als eine Pelletiermaschiene ausgebildet sein. Alternativ zur Pelletierung kann das Gemisch durch Pressen auch in ein Zwischenprodukt mit länglicher Form, beispielsweise eine Nudelform umgewandelt werden. Die Dicke des geformten Zwischenproduktes beträgt vorzugsweise zwischen 1-10 mm, besonders bevorzugt 2 bis 5 mm. Dabei ist aufgrund der Formgebung des Zwischenproduktes während des Fermentationsprozesses eine verbesserte Durchlüftung gegenüber Anlagen ohne eine entsprechende Vorrichtung 3 gegeben, wodurch die Effektivität der Schimmelpilzkulturen steigt.

Dieses geformte, beispielsweise pelletierte Zwischenprodukt gelangt im Anschluss an den Pressvorgang in eine Fermenteranlage. Für den kontinuierlichen Betrieb muss allerdings ein stetiger Abtransport des Zwischenproduktes von der Vorrichtung 3 gewährleistet werden. Im vorliegenden Fall erfolgt dieser Abtransport durch eine Transporteinrichtung 4, welche im vorliegenden Fall als Förderband ausgestaltet ist.

Weiterhin weist die Fermenteranlage eine Klimakammer 5 auf. In diesem Bereich der Fermentationsanlage werden die Bedingungen für eine Fermentation gezielt eingestellt. Bevorzugte Bedingungen für eine Fermentation sind dabei Temperaturen von 28-35 °C und eine Luftfeuchte von 80-100 %. Die Fermenteranlage kann sowohl im Chargenverfahren als auch im kontinuierlichen Verfahren betrieben werden. Das kontinuierliche Verfahren hat sich dabei als besonders günstig für die Verarbeitung erwiesen. Daher erfolgt die Fermentation in der Klimakammer vorzugsweise bei einer kontinuierlichen Vorschubgeschwindigkeit pro Meter Förderbandlänge von 0,1 bis 1 m/Tag. So kann in einem kontinuierlichen Verfahren bei entsprechender Dimensionierung der Klimakammer 5 und des Förderbandes 4 eine kontinuierliche Fermentation von 1-10 Tagen erreicht werden. Die obengenannte Transportgeschwindigkeit ermöglicht dabei einerseits ein hochwertiges Produkt, andererseits ein kontinuierliches Produktionsverfahren um eine effizientere Massenproduktion von Futtermitteln zu ermöglichen. Längere Fermentationszeiten werten dabei das Produkt auf.

Optional wird der Produktstrom oder die Produktcharge von der Fermenteranlage zu einer Vorrichtung zur Teilung der Gesamtproduktmenge zugeführt. Diese Vorrichtung trennt eine definierte Teilmenge des Produktstroms oder der Produktcharge ab und führt diese definierte Teilmenge in die Mischvorrichtung zurück. Dies erfolgt über eine Rückführung 6. Durch die Rückführung der definierten Teilmenge des Produktes kann der stete Zusatz von Schimmelpilz-Starterkulturen verringert oder sogar eingestellt werden, so dass die Schimmelpilz-Starterkulturen nur einmalig bei in Betriebnahme der erfindungsgemäßen Anlage zugeführt werden müssen.

Der verbleibende Produktstrom oder Produktcharge P wird nach dem Abtrennen der Teilmenge aus der Anlage abgeführt und kann in weiteren Bearbeitungsschritten zu Futterpellets zum Abtransport verpresst werden.

Das Produkt, welches im Anschluss den Fermenter verlässt, ist ein proteinreiches pflanzliches Produkt P. Dieses kann ggf. durch eine weitere hier nicht abgebildete Vorrichtung zur Formgebung in eine komprimierte Form, beispielsweise Pellets überführt werden.

Optional kann die Anlage zudem über Mittel zur Rückführung 6 eines Teilstroms des proteinreichem pflanzlichen Produktes verfügen, welche es ermöglichen das mit Kulturen angereicherte Produkt in die Mischvorrichtung 2 zurückzuführen. Dadurch werden dem Presskuchen weitere Mikroorganismen, vorzugsweise Hefen, insbesondere Saccharomyces cerevisiae, die während der Fermentation Vitamine und/oder Methionin bilden, zugeführt.

Nachfolgend wird ein erfindungsgemäßes Verfahren anhand eines Ausführungsbeispiels näher erläutert:

Das Verfahren setzt bei der Verarbeitung eines Rapspresskuchens nach der Gewinnung von Rapsöl durch den Vorgang der Ölpressung an. Dabei kann neben einem oder mehreren Pressvorgängen auch ein Extraktionsvorgang mit einem organischen Lösungsmittel vorgesehen sein, durch den der Ölgehalt des Presskuchens weiter verringert wird. Als organisches Lösungsmittel ist dabei z.B. Hexan geeignet. Der Presskuchen kann so zu Beginn des im Folgenden dargestellten Verfahrens einen geringen Ölgehalt von beispielsweise unter 3% aufweisen und damit praktisch fettfrei sein. Es ist auch möglich, den Extraktionsschritt mit dem organischen Lösungsmittel zu einem späteren Zeitpunkt des folgenden Verfahrens durchzuführen, beispielsweise nach dem nachfolgend beschriebenen Zerkleinerungsschritt.

Im Anschluss an die Ölpressung kann in einem ersten optionalen Schritt des Verfahrens eine Pasteurisierung des Rapspresskuchens erfolgen. Dadurch werden Mikroorganismen, insbesondere auch Pilzsporen und dergleichen, sowie andere unerwünschte Organismen, welche die Fermentation negativ beeinflussen könnten, abgetötet. Zudem wird die Lagerfähigkeit des hergestellten Produktes erhöht. Für eine Pasteurisierung können sowohl Infrarotbestrahlung, Dampf oder Mikrowellen eingesetzt werden. Die Pasteurisierung erfolgt bei Temperaturen von 50-80°C und Einwirkzeiten zwischen 1 Sekunde bis zu 4 Minuten.

Für einen besonders energieeffizienten Betrieb der Anlage kann auch die Restwärme, welche bei einem nachfolgenden Komprimierungs- bzw. Formgebungsschritt freigesetzt wird, genutzt werden.

In einem sich daran anschließenden Schritt wird der Presskuchen zunächst für die Fermentation vorbereitet. Dabei wird der Presskuchen zunächst zerkleinert. Dies kann in einer Zerkleinerungseinrichtung erfolgen.

Nach dem Zerkleinern wird der Presskuchen in einer Mischvorrichtung durch Zusatz von Wasser angefeuchtet. Die Befeuchtung kann so erfolgen, dass sich ein Bereich von A_{w} = 0,6 bis A_{w} = 0,96 ergibt.

Anschließend kann optional eine weitere Pasteurisierung erfolgen. Dabei ist es wahlweise möglich, die letzte Stufe des Pressvorgangs und die dort auftretende Temperaturerhöhung, aber auch ein anderes Verfahren, wie Infrarot, Mikrowellen oder Dampf für die Pasteurisierung unter analogen Bedingung wie bei der zuvor beschriebenen Pasteurisierung, bei Temperaturen von 50-80 °C und Einwirkzeiten zwischen 1 Sekunde bis zu 4 min, zu nutzen.

Schließlich wird der zerkleinerte, angefeuchtete Presskuchen auf einen festen pH-Wert, vorzugsweise zwischen pH 3,5 bis pH 5,0 eingestellt. Nach diesen Schritten ist die Einstellung des Presskuchens als Substrat abgeschlossen.

Als Schimmelpilz-Kultur für die Fermentation wird vorzugsweise ein Schimmelpilz der Gattung Rhizopus, insbesondere Rhizopus microsporus var. oligosporus (Stammanmeldungsnummer CBS 338.62 oder NRRL 2710) verwendet. Hier ist neben dem Einsatz besonders produzierter Starterkulturen besonders bevorzugt auch eine Einmischung einer Teilmenge des fertigen Materials in den frischen Rohstoff mit einem Anteil zwischen 1 Gew.-% bis 30 Gew.-% möglich.

Weiterhin ist der Zusatz von einer weiteren Starterkultur, vorzugsweise von Hefen, besonders bevorzugt von Candida utilis, Candida tropicalis, Yarrowia lipolytica, Kluyveromyces lactis und/oder insbesondere Saccharomyces cerevisiae, zur Anreicherung des Produktes mit Vitaminen der B-Gruppe und/oder mit Methionin, möglich.

Der zerkleinerte und angefeuchtete Presskuchen mit eingestelltem pH-Wert wird nach dem Zusatz von Schimmelpilzkulturen im Rahmen dieser Anmeldung als Zwischenprodukt bezeichnet.

Die Schritte des Zerkleinerns, Anfeuchtens und Einstellens des pH-Wertes können gegebenenfalls auch in ein und demselben Schritt erfolgen, indem der Presskuchen durch ein Rührwerk zerkleinert und durch Zugabe einer Säure sowohl angefeuchtet als auch auf einen definierten pH-Wert voreingestellt wird.

Vor einer Fermentation des Zwischenproduktes kann besonders vorteilhaft eine Formgebung erfolgen, so dass das Zwischenprodukt in eine Pellet- oder Nudelform (Fussilli und andere Formen) gepresst werden. Diese Formgebung dient der Durchlüftung des Zwischenproduktes während der Fermentation, wodurch die Fermentationszeiten verkürzt werden können oder die Qualität des Produktes durch intensivere Fermentation zunimmt, im Vergleich zu einem Verfahren in welchem keine Formgebung des Zwischenproduktes erfolgt. Eine besonders vorteilhafte Durchlüftung ergab dabei ein Zwischenprodukt, bei dem die Pellet oder Nudelform im statistischen Mittel eine Dicke zwischen 1 bis 10 mm, vorzugsweise 2 bis 5 mm aufwies.

Anschließend erfolgt die Fermentation. Diese wird bei Temperaturen zwischen 25-35 °C, vorzugsweise zwischen 28-35 °C und unter aeroben Bedingungen durchgeführt. Die Luft bei der Oberflächenfermentation kann der Umgebung entnommen oder zwangsweise zugeführt werden. Die Schichtdicke des Zwischenproduktes bei der Fermentation wird vorzugsweise auf Werte zwischen 2 cm und 10 cm eingestellt, wobei die Durchlüftung während der Fermentation durch eine Formgebung des Zwischenproduktes verbessert werden kann.

Im Anschluss an die Fermentation kann vorzugsweise eine weitere Pasteurisierung und ggf. eine Trocknung erfolgen.

Das Fermentationsprodukt kann jetzt direkt, d. h. frisch verarbeitet werden. Alternativ ist es möglich, das Fermentationsprodukt durch Kühlung, Gefrieren oder Anwendung einer modifizierten oder kontrollierten Atmosphäre für einen späteren Gebrauch haltbar zu machen.

Die erfindungsgemäße Verfahren zur Herstellung von Futtermittel aus Rückständen der Rapsölgewinnung mit geschältem Raps wird vorzugsweise als kontinuierlicher Prozess ausgestaltet, es ist aber auch als chargenweiser Prozess durchführbar.

Das erfindungsgemäße Verfahren zur Herstellung eines proteinreichen fettarmen pflanzlichen Produktes eignet sich besonders vorteilhaft für die Verarbeitung von Rückständen, die bei der Rapsölgewinnung erhalten werden. Diese können zerkleinert, angefeuchtet, ggf. pasteurisiert und auf einen vorgewählten pH-Bereich eingestellt werden, dann mit einem Schimmelpilz geimpft und danach bis zur Bildung einer Schimmelpilzschicht fermentiert werden.

Durch die Fermentation von aus der Rapsölgewinnung erhaltenen Rückständen, wobei die Rückstände aus der Rapsölgewinnung mit geschältem Raps stammen, wird ein proteinreiches, pflanzliches fettarmes Produkt gewonnen, welches erstmals Verwendung als Tierfuttermittel oder als Tierfuttermittelkomponente findet. Dabei trägt die Beseitigung oder Verringerung von unerwünschten Stoffen wie Glucosinolaten, Rohfasern und ethanollöslichen Kohlenhydraten, insbesondere von Oligosacchariden, durch Fermentation vorteilhaft zu einer Erhöhung des Rapsanteils im Futter bei und verbessert dessen Eigenschaften als Tierfutter oder Tierfutterkomponente.

## Patentansprüche

1. Verwendung eines proteinreichen, pflanzlichen fettarmen Produktes, welches durch Fermentation aus von einer Rapsölgewinnung erhaltenen Rückständen gewonnen wird, wobei die Rückstände aus der Rapsölgewinnung vorzugsweise aus geschältem Raps stammen, als Tierfuttermittel oder Tierfuttermittelkomponente.

2. Verwendung nach Anspruch 1, **dadurch gekennzeichnet, dass** das Produkt einen Glucosinolatgehalt von ≤ 2 mmol/kg Trockenmasse, einen Rohfasergehalt von ≤ 8 Gew.% Trockenmasse und einen verringerten Gehalt an ethanollöslichen Kohlenhydraten von ≤ 10 g/kg Trockenmasse aufweist.

3. Anlage zur kontinuierlichen Herstellung eines proteinreichen fettarmen pflanzlichen Produktes aus einem Presskuchen, welche eine Fermenteranlage aufweist, **dadurch gekennzeichnet, dass** die Fermenteranlage eine Transporteinrichtung (4) für einen Transport eines Zwischenproduktes während der Fermentation aufweist und eine Klimakammer (5) zur Fermentation.

4. Anlage nach Anspruch 3, **dadurch gekennzeichnet, dass** die Anlage eine Mischvorrichtung (2) für die Zugabe von schimmelpilzhaltigem Material zum Presskuchen unter Bildung eines Zwischenproduktes aufweist.

5. Anlage nach Anspruch 3 oder 4, **dadurch gekennzeichnet, dass** die Anlage eine Vorrichtung (3) mit Mitteln zur Komprimierung und/oder Formgebung eines zu fermentierenden Zwischenproduktes aufweist.

6. Anlage nach einem der vorhergehenden Ansprüche, **dadurch gekennzeichnet, dass** die Anlage Rückführmittel (6) aufweist, zum Rückführen einer Teilmenge des Produktes zur Mischvorrichtung (2).

7. Anlage nach einem der vorhergehenden Ansprüche, **dadurch gekennzeichnet, dass** die Transporteinrichtung (4) eine kontinuierliche Vorschubgeschwindigkeit pro Meter Förderbandlänge von vorzugsweise 0,1-1 m pro Tag aufweist.

8. Verfahren zur Herstellung eines proteinreichen fettarmen pflanzlichen Produktes aus einem Presskuchen, mit folgenden Schritten:
a) Zerkleinern des Presskuchens;
b) Anfeuchten des Presskuchens;
c) Einstellen des pH-Wertes des Presskuchens;
d) Animpfen des Presskuchens mit einer Schimmelpilzkultur unter Bildung eines Zwischenproduktes; und
e) Fermentieren des Zwischenproduktes unter Bildung des Produktes, **dadurch gekennzeichnet, dass** nach der Bildung des Zwischenproduktes und vor dem Fermentieren eine Formgebung des Zwischenproduktes erfolgt.

9. Verfahren nach Anspruch 8, **dadurch gekennzeichnet, dass** die Formgebung des Zwischenprodukts in der Ausbildung einer Pellet- oder Nudelform erfolgt.

10. Verfahren nach einem der vorhergehenden Ansprüche, **dadurch gekennzeichnet, dass** vor dem Animpfen des Presskuchens eine Pasteurisierung des Presskuchens erfolgt.

11. Verfahren nach einem der vorhergehenden Ansprüche, **dadurch gekennzeichnet, dass** nach dem Fermentieren ein Rückführen einer Teilmenge des Produktes zum Animpfen des Presskuchens erfolgt, wobei die Teilmenge etwa 1-30 Gew.-% der Menge an Produkt nach der Fermentation umfasst.

12. Verfahren nach einem der vorhergehenden Ansprüche, **dadurch gekennzeichnet, dass** vor der Fermentation ein Zusatz einer weiteren Starterkultur zur Anreicherung des Produktes mit Vitaminen der Gruppe B und/oder Methionin erfolgt.

13. Verfahren nach einem der vorhergehenden Ansprüche, **dadurch gekennzeichnet, dass** durch die Fermentation eine Verringerung des Glucosinolatgehaltes gegenüber dem Glucosinolatgehalt des Presskuchens um zumindest 87 % erfolgt.

14. Verfahren nach einem der vorhergehenden Ansprüche, **dadurch gekennzeichnet, dass** durch die Fermentation eine Verringerung des Rohfasergehaltes gegenüber dem Rohfasergehalt des Presskuchens um zumindest 32 % erfolgt.

15. Verfahren nach einem der vorhergehenden Ansprüche, **dadurch gekennzeichnet, dass** durch die Fermentation eine Verringerung des Gehaltes an ethanollöslichen Kohlenhydraten gegenüber dem Gehalt an ethanollöslichen Kohlenhydraten des Presskuchens um zumindest als 90 % erfolgt.

16. Verfahren nach einem der vorhergehenden Ansprüche, **dadurch gekennzeichnet, dass** ein Ölgehalt des Presskuchens vor oder nach dem Schritt des Zerkleinerns durch einen Extraktionsvorgang mit einem organischen Lösungsmittel, insbesondere Hexan, verringert wird.
